(19) Europäisches Patentamt
European Patent Office
Office européen des brevets

(11) Publication number: 0 116 255
A2

(12) # EUROPEAN PATENT APPLICATION

(21) Application number: 83440061.6

(22) Date of filing: 06.12.83

(51) Int. Cl.³: C 07 D 211/42
C 07 D 211/46, A 61 K 31/445

(30) Priority: 10.12.82 GB 8235295

(43) Date of publication of application: 22.08.84 Bulletin 84/34

(84) Designated Contracting States: AT BE CH DE FR GB IT LI LU NL SE

(71) Applicant: MERRELL TORAUDE ET COMPAGNIE
16 Rue d'Ankara
F-67084 Strasbourg(FR)

(72) Inventor: Fozard, John R.
22 rue Vermeer
F-67200 Strasbourg-Elsau(FR)

(72) Inventor: Gittos, Maurice W.
16 rue Andre Malraux Plobsheim
F-67400 Illkirch-Graffenstaden(FR)

(74) Representative: Vossius Vossius Tauchner Heunemann Rauh
Siebertstrasse 4 P.O. Box 86 07 67
D-8000 München 86(DE)

(54) **Treatment of migraine with N-alkyl-piperidinyl benzoate derivatives.**

(57) Migraine is treated with an N-alkyl-(3 or 4)-piperidinyl benzoate compound of general Formula I:-

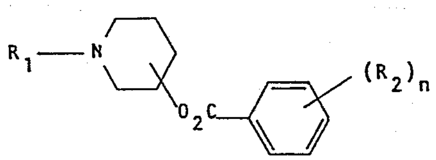

(I)

wherein

the benzoyloxy group is at the 3 or 4 position of the piperidine ring:

$n$ is 0, or an integer from 1 to 5;

$R_1$ represents $C_1$-$C_4$ alkyl; and

each $R_2$ represents $C_1$-$C_4$ alkyl, $C_1$-$C_4$ alkoxy, or halogen, provided that, when $n$ is 2, the groups represented by $R_2$ can be either the same or different, and, when $n$ is 3, 4, or 5, the groups represented by $R_2$ must be the same,

or a pharmaceutically acceptable salt thereof.

EP 0 116 255 A2

Croydon Printing Company Ltd.

PATENTANWÄLTE
SIEBERTSTR. 4, 8000 MÜNCHEN 80
TEL (089) 47 40 75

Our ref.: S 737 EP
Case: C-31,387 EP

0116255

MERRELL-TORAUDE ET COMPAGNIE
Stasbourg, France

# TREATMENT OF MIGRAINE WITH N-ALKYL-PIPERIDINYL BENZOATE DERIVATIVES

The invention relates to certain N-alkyl -(3 or 4)- piperidinyl benzoate derivatives useful for the treatment of migraine. The invention includes the novel compounds *per se*, pharmaceutical compositions containing said compounds, and methods for using said compounds for treating migraine.

Acute attacks of migraine are usually treated with a peripheral vasoconstrictor, such as ergotamine, which may be co-administered with caffeine, and dihydroergotamine; an antipyretic analgesic, such as acetylsalicylic acid or p-acetylaminophenol; and/or an anti-emetic such as cyclizine, metoclopramide and thiethylperazine. It has also been reported (J.B. Hughes; *Med. J. Aust.* 2, No. 17, 580, (1977) that immediate relief of acute migraine attack can be obtained by slow intravenous injection of metoclopramide (10 mg).

It is believed that 5-hydroxytryptamine (5-HT) is the naturally occurring substance most likely to play a role in the pathophysiology of migraine. Increased amounts of 5-HT and its metabolite 5-hydroxyindoleacetic acid are excreted in the urine during most

attacks. Further, plasma and platelet 5-HT concentrations fall rapidly at the onset of an attack and remain low whilst the headache persists. Moreover, attacks of migraine have been clearly associated with periods of thrombocytopaenia in certain patients. It has been proposed that compounds which block the activity of 5-HT would be of use in the treatment of migraine (J.R. Fozard, International Headache Congress 1980) reported in Advances in Neurology, Vol 33, Raven Press, New York 1982 pp. 295-307).

The known migraine prophylactic drugs methysergide, propranolol, amitriptyline, and chlorpromazine have widely different pharmacological activities but are all 5-HT D-receptor antagonists at the doses used clinically for the treatment of migraine. Metoclopramide is a potent 5-HT M-receptor antagonist and it has been proposed (J.R. Fozard supra) that blockade of the M-receptor present on afferent sensory neurones affords symptomatic relief in an acute migraine attack.

The potency as 5-HT M-receptor antagonists of (-)-cocaine and some related compounds has been reported (J.R. Fozard et al, Eur. J. Pharmacol., 59, 195-210 (1970)) but, with the exceptions of nor(-)cocaine and tropyl benzoate, none are as potent as metoclopramide.

The $pA_2$ 5-HT values reported for nor(-)cocaine and tropyl benzoate are 7.7 and 7.2 respectively, whilst the $pA_2$ 5-HT value determined for metoclopramide by the same procedure is 7.2 (J.R. Fozard _et al_. _Eur. J. Pharmacol_., 49, 109-112 (1978)).

In its first aspect, the present invention provides the N-alkyl-(3 or 4)-piperidinyl benzoate compounds of general Formula I:-

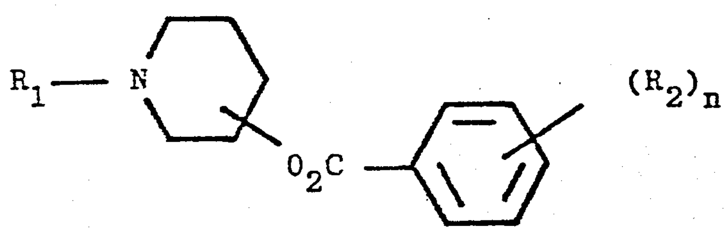

(I)

wherein:

the benzoyloxy group is at the 3 or 4 position of the piperidine ring;

_n_ is 0, or an integer from 1 to 5;

$R_1$ represents $C_1$-$C_4$ alkyl; and

each $R_2$ represents $C_1$-$C_4$ alkyl, $C_1$-$C_4$ alkoxy, or halogen, provided that, when _n_ is 2, the groups represented by $R_2$ can be either the same or different, and, when _n_ is 3, 4, or 5, the groups represented by $R_2$ must be the same,

or a pharmaceutically acceptable salt thereof.

In a second aspect, the invention provides pharmaceutical compositions for the effective relief of migraine comprising a compound of general Formula I

in admixture or otherwise associated with a pharmaceutically acceptable diluent or carrier. Preferably, the said compositions are in unit dose form containing 5 to 1000 mg per unit dose. Usually, said compositions will contain 10 to 500 mg, especially 30 to 300 mg, per unit dose.

According to a third aspect of the invention, there are provided compounds of Formula I for use in the treatment of migraine.

In a fourth aspect, the invention provides a method of treating migraine which comprises administering to a patient suffering migraine, an effective migraine relieving amount of a compound of general Formula I. Said amount usually will be in the range 0.1 mg/kg to 10 mg/kg, especially 0.3 mg/kg to 30 mg/kg. It is also contemplated that the compounds of general Formula I can be used in the prophylaxis of migraine by administering to a patient at risk of migraine an effective migraine-prophylatic amount of the compound.

It will be apparent to those skilled in the art that the phenyl ring of the benzoyloxy group in Formula I is unsubstituted, monosubstituted, disubstituted, trisubstituted, tetrasubstituted, or pentasubstituted as the value of <u>n</u> varies, respectively, from 0 to 5. When <u>n</u> is 1, the

substituent represented by $R_2$ can be located at any available position on the phenyl ring (i.e. the 2-, 3-, or 4-position. When n is 2, each of the two substituents represented by $R_2$ can be different but preferably are the same, and they can be located at at any of the available positions on the phenyl ring (i.e. the 2,3-, 2,4-, 2,5-, 2,6-, 3,4-, or 3,5-positions). When n is 3, each of the three substituents represented by $R_2$ must be the same, and they can be located at any of the available positions of the phenyl ring (i.e. the 3,4,5-, 2,3,4-, 2,4,6-, 2,3,5-, 2,3,6-, or 2,4,5- positions). When n is 4, each of the four substituents represented by $R_2$ must be the same, and they can be located at any of the available positions on the phenyl ring (i.e. the 2,3,4,5-, 2,4,5,6-, or 2,3,5,6-positions). When n is 5, each of the five substituents represented by $R_2$ must be the same, and they are located at the available positions of the phenyl ring (i.e. the 2,3,4,5,6-positions).

It presently is preferred that n is 0 to 3, especially 2 and then particularly with both substituents represented by $R_2$ being the same and in the 3,5-positions of the phenyl ring.

In one subgeneric aspect of the invention, there

are provided N-alkyl-3-piperidinyl benzoate compounds of the following Formula I<u>a</u>:-

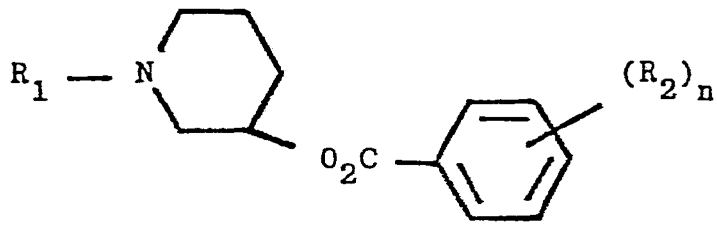

(I<u>a</u>)

wherein $R_1$, <u>n</u> and $R_2$ are as defined in connection with Formula I.

Subclasses of compounds within the scope of Formula I<u>a</u> are:-

N-alkyl-3-piperidinyl-unsubstituted benzoates

N-alkyl-3-piperidinyl-monosubstituted benzoates

N-alkyl-3-piperidinyl-disubstituted benzoates

N-alkyl-3-piperidinyl-trisubstituted benzoates

N-alkyl-3-piperidinyl-tetrasubstituted benzoates

N-alkyl-3-piperidinyl-pentasubstituted benzoates

In another and presently preferred subgeneric aspect of the present invention, there are provided N-alkyl-4-piperidinyl- benzoate compounds of the following general Formula I<u>b</u>:-

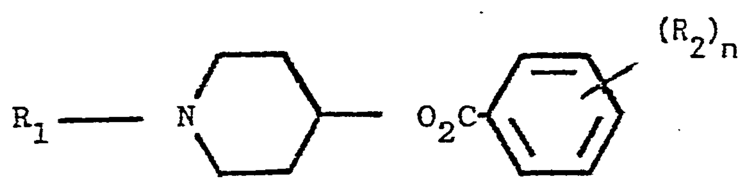

(I<u>b</u>)

wherein $R_1$, n and $R_2$ are as defined in connection with Formula I.

Subclasses of compounds within the scope of Formula Ib are:-

N-alkyl-4-piperidinyl-unsubstituted benzoates
N-alkyl-4-piperidinyl-monosubstituted benzoates
N-alkyl-4-piperidinyl-disubstituted benzoates
N-alkyl-4-piperidinyl-trisubstituted benzoates
N-alkyl-4-piperidinyl-tetrasubstituted benzoates
N-alkyl-4-piperidinyl-pentasubstituted benzoates

Examples of $C_1$-$C_4$ alkyl groups which can be represented by $R_1$ or $R_2$ are methyl, ethyl, n-propyl, n-butoxy and iso-propyl with ethyl and, especially, methyl being preferred.

Examples of $C_1$-$C_4$ alkoxy groups which can be represented by $R_2$ are methoxy, ethoxy, n-propoxy, n-butoxy, and iso-propoxy, with ethoxy and, especially, methoxy being preferred.

The halogens which can be represented by $R_2$ are bromine, chlorine, fluorine, and iodine, with bromine, fluorine and, especially, chlorine being preferred.

Illustrative examples of the compounds of Formulae Ia and Ib are those wherein $R_1$ is methyl and the phenyl ring is unsubstituted or substituted as follows:-

2-methyl,
3-methyl,
4-methyl,
2-methoxy,
3-methoxy,
4-methoxy,
2-chloro,
3-chloro,
4-chloro,
2,3-dimethyl,
2,4-dimethyl,
2,5-dimethyl,
2,6-dimethyl,
3,4-dimethyl,
3,5-dimethyl,
2,3-dimethoxy,
2,4-dimethoxy,
2,5-dimethoxy,
2,6-dimethoxy,
3,4-dimethoxy,
3,5-dimethoxy,
2,3-dichloro,
2,4-dichloro,
2,5-dichloro,
2,6-dichloro,
3,4-dichloro,
3,5-dichloro,

3,4,5-trimethyl,

3,4,5-trimethoxy, and

3,4,5-trichloro.

The compounds of Formula I block the M-receptors for 5-hydroxytryptamine (5-HT) on afferent sensory neurones, certain of which subserve the transmission of pain. As explained above, the blocking of such M-receptors is believed to be a mechanism by which the symptoms of migraine can be relieved. Accordingly, the compounds of Formula I are useful in the treatment of migraine when administered in amounts sufficient to effectively block the said M-receptors.

The activity of the compounds against 5-HT can be assessed by determining their $pA_2$ values in the isolated rabbit heart as described by J.R. Fozard *et al* *Europ. J. Pharmacol.* 59, 195-210 (1979). In the method described the molar concentration of antagonist which reduces the effects of twice the ED50 of 5-HT to that of the ED50 in the absence of antagonist is determined. The $pA_2$ value is the negative logarithm of said molar concentrations. In general terms, the higher the $pA_2$ value the more potent is the compound.

The $pA_2$ 5-HT values of N-methyl-4-piperidinyl 3',5'-dimethylbenzoate and N-methyl-3-piperidinyl 3',5'-dimethylbenzoate are 8.1 and 7.3, respectively.

The activity of the compounds of Formula I against 5-HT can be assessed *in vivo* by measurement of the effect of the compound on the Von Bezold-Jarisch Reflex induced by 5-HT injected intravenously into the rat (see A.S. Paintal, Physiol. Rev. 53 (159-227), 1973). The transient cardiac slowing results arises from an increased efferent vagus activity arising from stimulation by 5-HT of sensory afferent fibres in and around the heart.

The compounds of Formula I are believed to be highly selective in their action against 5-HT M-receptor. Their potency against other 5-HT receptors and other spasmogens, in particular oxytocin, acetylcholine, histamine and calcium, is believed to be appreciably lower than that against 5-HT M-receptors. Accordingly, their use in the treatment of migraine should be essentially without any side effects.

The compounds of Formula I can be administered in various manners to achieve the desired effect. The compounds can be administered alone or in the form of pharmaceutical preparations to the patient being treated either orally or parenterally, for example, subcutaneously or intravenously. The amount of compound administered will vary and can be any effective migraine-relieving amount. Depending upon

the patient and the mode of administration, the quantity of compound administered may vary over a wide range to provide from about 0.1 mg/kg to about 100 mg/kg, usually 0.3 to 30 mg/kg, of body weight of the patient per dose. Unit doses of these compounds can contain, for example, from about 5 mg to 1000 mg, usually 10 to 500 mg and preferably 30 to 300 mg, of the compound and may be administered, for example, from 1 to 4 times daily.

The term "unit dosage form" is used herein to mean a single or multiple dose form containing a quantity of the active ingredient in admixture with or otherwise in association with the diluent or carrier, said quantity being such that one or more predetermined units are normally required for a single therapeutic administration. In the case of multiple dose forms such as liquids or scored tablets, said predetermined unit will be one fraction, such as a 5 ml (teaspoon) quantity of a liquid or a half or quarter of a scored tablet, of the multiple dose form.

In the composition aspect of the invention there are provided pharmaceutical formulations in which form the active compounds of the invention will normally be utilized. Such formulations are prepared in a manner well known *per se* in the pharmaceutical art and

usually comprise at least one active compound of the invention in admixture or otherwise in association with a pharmaceutically acceptable carrier or diluent therefor. For making those formulations the active ingredient will usually be mixed with a carrier, or diluted by a diluent, or enclosed or encapsulated in a capsule, sachet, cachet, paper or other container. A carrier or diluent may be solid, semi-solid or liquid material which serves as a vehicle, excipient or medium for the active ingredient. Suitable carriers or diluents are well known per se.

The formulations of the invention may be adapted for enteral or parenteral use and may be administered to the patient in the form of tablets, capsules, suppositories, solutions, suspensions or the like.

In the specific examples included hereinbelow illustrative examples of suitable pharmaceutical formulations are described.

The N-alkyl-piperidinyl benzoate derivatives of Formula I can be used in migraine therapy with antimigraine drugs having different modes of action. Such drugs include those used prophylactically, such as barbiturates, diazepam, chlorpromazine, amitriptyline, propranolol, methysergide, pizotifen, cyproheptadine, dihydroergotamine, and clonidine, and

those used in the acute attack, such as vasoconstrictor agents, e.g. ergotamine and dihydroergotamine, analgaesic/antiinflammatory agents, e.g. aspirin, paracetamol and indomethacin, or antinauseants, e.g. cyclizine, metoclopramide, and triethylperazine (see J.R. Fozard, J. Pharm. Pharmacol. 27, 297-321 (1975); J.R. Saper, J. Amer. Med. Assoc. 239, 480-484 (1978)); J.R. Fozard, supra.) As an example, compounds of general Formula 1 would be beneficial in combination with aspirin 300-1200 mg or methysergide, 2-6 mg given daily.

The compounds of general Formula I can be prepared in manner known per se from an N-alkyl-(3 or 4)-piperidinol of general Formula II:

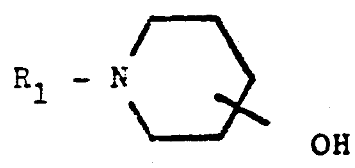

(II)

wherein $R_1$ is $C_1$-$C_4$ alkyl, and a benzoic acid halide of general Formula III:

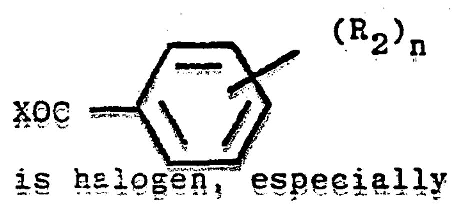

III

wherein X is halogen, especially chlorine, and n and $R_2$ have the meanings defined in connection with Formula I.

The starting materials of Formulae II, and III are either known compounds or can be made by known methods, or by modifications of known methods, from known compounds.

The reaction usually will be carried out by stirring the acid halide and N-alkyl-piperidinol in an aprotic solvent, preferably methylene chloride or acetonitrile, at ambient temperature. The solvent is evaporated off, usually under reduced pressure, after about 2 to 16 hours and water added to the residue, followed by aqueous base, such as sodium or potassium carbonate, which does not hydrolyse the ester, to render the aqueous product solution alkaline. Subsequently the desired free base is extracted with a suitable organic solvent such as, for example, diethyl ether, ethylacetate or methylene chloride. The organic solution is then washed with water to remove excess piperidinol and dried. The organic solvent is evaporated off and the free base recrystallized from, for example, aqueous methanol. Alternatively, the crude free base can be converted into an acid addition salt, preferably hydrochloride, by addition of an ethereal solution of the acid. The acid salt is recrystallized from, for example ethanol or isopropanol.

As mentioned previously, the compounds of Formula I can be used in the form of their pharmaceutically acceptable acid addition salts.

The pharmaceutically acceptable acid addition salts can be non-toxic addition salts with suitable acids, such as those with inorganic acids, for example hydrochloric, hydrobromic, nitric, sulfuric or phosphoric acids, or with organic acids, such as organic carboxylic acids, for example, glycollic, maleic, hydroxymaleic, malic, tartaric, citric, salicylic, o-acetyloxybenzoic, nicotinic or isonicotinic, or organic sulphonic acids, for example methane sulphonic, ethane sulphonic, 2-hydroxyethane sulphonic, toluene-p-sulphonic, or naphthalene-2-sulphonic acids.

Apart from pharmaceutically acceptable acid addition salts, other acid addition salts, such as for example, those with picric or oxalic acid, may be serve as intermediates in the purification of the compounds or in the preparation of other, for example, pharmaceutically acceptable, acid addition salts, or are useful for identification or characterisation of the bases.

An acid addition salt may be converted into the free compound according to known methods, for example, by treating it with a base, such as with a metal

hydroxide or alkoxide, for example an alkali or alkaline earth metal hydroxide, for example, lithium hydroxide, sodium hydroxide, potassium hydroxide or calcium hydroxide; with a metal carbonate, such as an alkali metal or an alkaline earth metal carbonate or hydrogen carbonate, for example, sodium, potassium or calcium carbonate or hydrogen carbonate; with trialkylamine; or with an anion exchange resin.

An acid addition salt may also be converted into another acid addition salt according to known methods; for example, a salt with an inorganic acid may be treated with a metal salt, for example a sodium, barium or silver salt, or an acid in a suitable diluent, in which a resulting inorganic salt is insoluble and is thus removed from the reaction medium. Acid addition salt may also be converted into another acid addition salt by treatment with an anion exchange preparation.

The invention is illustrated in the following non-limiting Examples.

EXAMPLE 1

N-Methyl-4-piperidinyl-3',5'-dimethylbenzoate

A mixture of 3,5-dimethylbenzoylchloride (1.65 g, 0.01 mole) and N-methyl-4-piperidinol (1.15 g, 0.01 mole) was stirred for 16 hours in acetonitrile (50 ml) at room temperature. The solvent was evaporated, and

the residue treated with water (50 ml) and saturated potassium carbonate solution (5 ml). The liberated oil was extracted into ether, the ether solution was washed several times with water, and the washed ether solution then dried over magnesium sulfate. Distillation of the dried ether solution gave a residue which was treated with anhydrous ether and etheral hydrogen chloride. Crystallization of the precipitate from ethanol afforded N-methyl-4-piperidinyl-3',5'-dimethylbenzoate, as the hydrochloride: m.p. 212-4°C.

EXAMPLE 2

N-Methyl-3-piperidinyl-3',5'-dimethylbenzoate

Following the procedure of Example 1, but using N-methyl-3-piperidinol (1.15 g, 0.01 mole) in place of N-methyl-4-piperidinol and oxalic acid instead of hydrogen chloride there was obtained, upon recrystallization from methanol, the title product in the form of its mono-oxalate salt: m.p. 176.5 - 177.5°C.

EXAMPLE 3

Antagonism of the von Bezold-Jarisch reflex evoked by 5-HT in the anaesthetised rat was measured for N-methyl-4-piperidinyl-3',5'-dimethylbenzoate (Compound A) and N-methyl-3-piperidinyl 3',5'-dimethylbenzoate (Compound B), using the following method:

Male Sprague-Dawley rats weighing 250-300 g are anaesthetized with urethane, 1.25 g/kg injected intraperitoneally and set up for recording blood pressure and heart rate as described in Fozard J.R *et al.*, J. Cardiovasc. Pharmacol. 2, 229-245 (1980). A submaximal dose of 5-HT (2 μg/kg) is given repeatedly into the cannulated jugular vein and changes in heart rate quantified. Antagonists are given intravenously and the doses required to just inhibit the response to 5-HT (threshold dose) or to inhibit the response to 5-HT by 50% (ED50) are determined.

The results obtained are set forth in Table I below in which the compounds are identified by the reference letters used above.

TABLE I

VON BEZOLD-JARISCH REFLEX ANTAGONISM

| COMPOUND | THRESHOLD DOSE (μg/kg) | ED50 (μg/kg) |
|---|---|---|
| A | | 136 ± 22 |
| B | | 510 ± 116 |

In a comparative test metoclopramide gave a threshold dose of 233.4 ± 66.3 μg/kg and an ED50 of 408.4 ± 80.9 μg/kg.

In the following Examples relating to pharmaceutical compositions, the term "active

compound" is used to indicate the compound N-methyl-4-piperidinyl-3',5'-dimethylbenzoate. This compound may be replaced in these compositions by any other compound of Formula I, for example by N-methyl-3-piperidinyl-3',5'-dimethylbenzoate. Adjustments in the amount of medicament may be necessary or desirable depending upon the degree of activity of the medicament as is well known in the art.

## EXAMPLE 4

An illustrative composition for hard gelatin capsules is as follows:-

| | | |
|---|---|---|
| (a) | active compound | 5 mg |
| (b) | talc | 5 mg |
| (c) | lactose | 90 mg |

The formulation is prepared by passing the dry powders of (a) and (b) through a fine mesh screen and mixing them well. The powder is then filled into hard gelatin capsules at a net fill of 100 mg per capsule.

## EXAMPLE 5

An illustrative composition for tablets is as follows:-

| | | |
|---|---|---|
| (a) | active compound | 5 mg |
| (b) | starch | 43 mg |
| (c) | lactose | 50 mg |
| (d) | magnesium stearate | 2 mg |

The granulation obtained upon mixing the lactose with the compound (a) and part of the starch and granulating with starch paste is dried, screened, and mixed with the magnesium stearate. the mixture is compressed into tables weighing 100 mg each.

EXAMPLE 6

An illustrative composition for an injectable suspension is the following 1 ml ampule for an intramuscular injection:-

| | | Weight per cent |
|---|---|---|
| (a) | active compound | 0.01 |
| (b) | polyvinylpyrrolidone | 0.5 |
| (c) | lecithin | 0.25 |
| (d) | water for injection to make | 100.0 |

The material (a) - (d) are mixed, homogenized, and filled into 1 ml ampules which are sealed and autoclaved 20 minutes at 121°C. Each ampule contains 1.0 mg per ml of compound (a).

EXAMPLE 7

| | mg/suppository |
|---|---|
| Active Compound | 5 |
| Oil of Theobroma (cocoa butter) | 995 |

The medicament is powdered and passed through a B.S. No. 100 Sieve and triturated with molten oil of theobroma at 45°C to form a smooth suspension. The mixture is well stirred and poured into moulds each of nominal 1G capacity, to produce suppositories.

CLAIMS:

1. An N-alkyl-(3 or 4)-piperidinyl benzoate derivative having the formula (I)

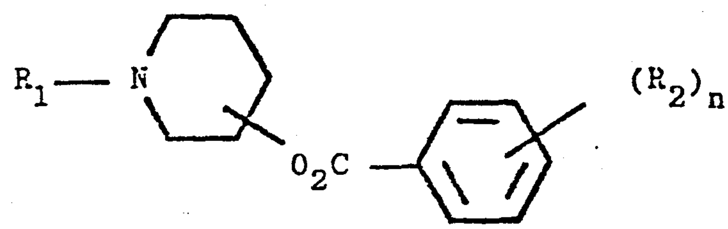

(I)

wherein

the benzoyloxy group is in the 3 or 4 position of the piperidine ring,

n is 0, or an integer from 1 to 5;

$R_1$ represents $C_1$-$C_4$ alkyl, and

$R_2$ represents $C_1$-$C_4$ alkyl, $C_1$-$C_4$ alkoxy or halogen, with the proviso that when n is 2, $R_2$ can be either the same or different, and when n is 3, 4 or 5 the $R_2$ groups must all be the same, or a pharmaceutically acceptable salt thereof.

2. An N-alkyl-3-piperidinyl benzoate compound having the following formula (I a)

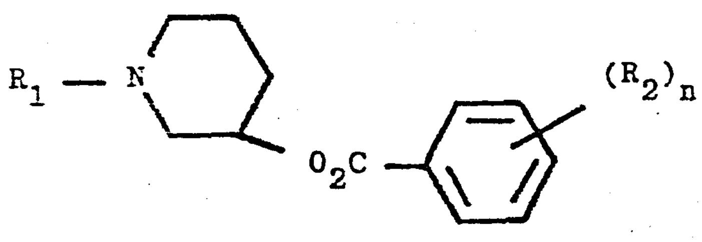

(Ia)

wherein $R_1$, n and $R_2$ are as defined in Claim 1, or a pharmaceutically acceptable salt thereof.

3. An N-alkyl-4-piperidinylbenzoate compound having the following general formula (I b)

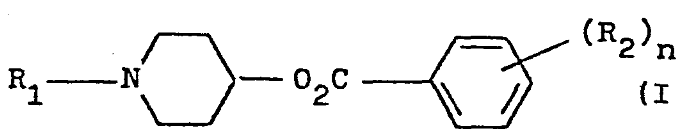

(I b)

wherein $R_1$, n and $R_2$ are as defined in Claim 1, or a pharmaceutically acceptable salt thereof.

4. A compound according to Claim 1 wherein n is 2.

5. A compound according to Claim 4 wherein the $R_2$ substituents are the same and are located at the 3,5-positions of the phenyl ring.

6. A compound according to Claim 1 wherein $R_1$ is methyl.

7. N-methyl-3-piperidinyl-3',5'-dimethylbenzoate or a pharmaceutically acceptable salt thereof.

8. N-methyl-4-piperidinyl-3',5'-dimethylbenzoate or a pharmaceutically acceptable salt thereof.

9. A pharmaceutical composition comprising a compound of Claim 1 in admixture or otherwise associated with a pharmaceutically acceptable diluent or carrier.

10. A pharmaceutical composition according to Claim 9 which contains from 30 to 300 mg of said compound per unit dose.

11. A process for preparing a compound of Claim 1 which comprises reacting an N-alkyl-(3 or 4)-piperidinol having the formula

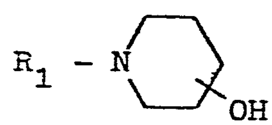

wherein $R_1$ is $C_1$-$C_4$ alkyl with a benzoic acid halide having the formula

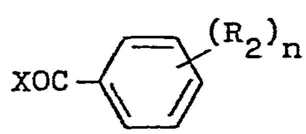

wherein X is halogen and n and $R_2$ are as defined in Claim 1.

12. A process according to Claim 11 wherein the reaction is conducted in an aprotic solvent at ambient temperature.

13. An N-alkyl-(3 or 4)-piperidinyl benzoate derivative having the formula

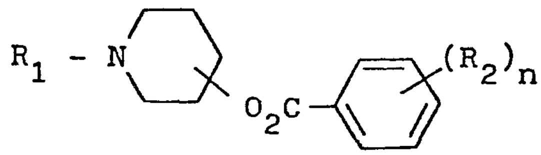

wherein the benzoyloxy group is in the 3 or 4 position of the piperidine ring,

n is 0, or an integer from 1 to 5;

$R_1$ represents $C_1$-$C_4$ alkyl, and

$R_2$ represents $C_1$-$C_4$ alkyl, $C_1$-$C_4$ alkoxy or halogen, with the proviso that when n is 2, $R_2$ can be either the same or different, and when n is 3, 4 or 5 the $R_2$ groups must all be the same, or a pharmaceutically acceptable salt thereof, for use in the treatment of migraine.